# EUROPÄISCHE PATENTSCHRIFT

(11) **EP 1 442 023 B1**
(45) Veröffentlichungstag und Bekanntmachung des Hinweises auf die Patenterteilung: **15.08.2007**
(21) Anmeldenummer: 02777305.0
(22) Anmeldetag: 11.10.2002
(51) Int. Cl.: C07D 235/20, A61K 31/4184, A61P 9/12

(54) **KRISTALLINES NATRIUMSALZ DES TELMISARTANS UND DESSEN VERWENDUNG ALS ANGIOTENSIN ANTAGONIST**
CRYSTALLINE SODIUM SALT OF TELMISARTAN AND THE USE OF THE SAME AS AN ANGIOTENSIN ANTAGONIST
SEL DE SODIUM CRISTALLISE DE TELMISARTAN ET SON UTILISATION COMME ANTAGONISTE DE L'ANGIOTENSINE

(30) Priorität: 31.10.2001 DE 10153737
(43) Veröffentlichungstag der Anmeldung: 04.08.2004
(73) Patentinhaber: Boehringer Ingelheim Pharma GmbH & Co.KG, 55216 Ingelheim am Rhein (DE)
(72) Erfinder: DONSBACH, Kai, 55595 Hargesheim (DE); HOF, Irmgard, 55218 Ingelheim Am Rhein (DE)
(86) Internationale Anmeldenummer: PCT/EP2002/011394
(87) Internationale Veröffentlichungsnummer: WO 2003/037876

(56) Entgegenhaltungen:
- EP-A- 0 502 314
- WO-A-00/43370

## Beschreibung

Die Erfindung betrifft ein kristallines Natriumsalz von 4'-[2-n-Propyl-4-methyl-6-(1-methylbenzimidazol-2-yl)benzimidazol-1-ylmethyl]biphenyl-2-carbonsäure (INN: Telmisartan), Verfahren zu dessen Herstellung, sowie dessen Verwendung zur Herstellung eines Arzneimittels.

### Hintergrund der Erfindung

Die Verbindung Telmisartan ist aus dem Europäischen Patent EP 502 314 B1 bekannt und weist die folgende chemische Struktur auf: 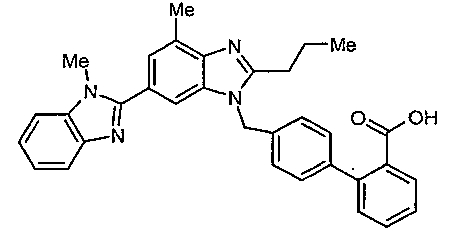 Telmisartan, sowie dessen physiologisch verträgliche Salze, besitzen wertvolle pharmakologische Eigenschaften. Telmisartan stellt einen Angiotensin-Antagonisten, insbesondere einen Angiotensin-II-Antagonisten dar, der aufgrund seiner pharmakologischen Eigenschaften bespielsweise zur Behandlung der Hypertonie und Herzinsuffizienz, zur Behandlung ischämischer peripherer Durchblutungsstörungen, der myokardialen lschämie (Angina), zur Prävention der Herzinsuffizienzprogression nach Myokard-Infarkt, zur Behandlung der diabetischen Neuropathie, des Glaukoms, von gastrointestinalen Erkrankungen sowie von Blasenerkrankungen Verwendung finden kann. Weitere mögliche Therapiegebiete sind der EP 502314 B1 zu entnehmen, auf die an dieser Stelle inhaltlich Bezug genommen wird.

Telmisartan ist unter dem Handelsnamen Micardis^{®} im Handel erhältlich. Ausgehend von der freien Säure des Telmisartans wird die Darreichungsform, mittels derer Telmisartan vermarktet wird, über ein aufwendiges Sprühtrocknungsverfahren hergestellt. Aufgrund der geringen Löslichkeit der freien Säure sind weniger aufwendige Verfahren zur Herstellung einer alternativen Darreichungsform nur schwer realisierbar.

Es ist Aufgabe der vorliegenden Erfindung, Telmisartan in einer Form bereitzustellen, die die Darstellung einer Darreichungsform dieses Wirkstoffs in weniger aufwendiger Form erlaubt. Hierbei muß berücksichtigt werden, daß sich generell die Herstellung einer einen Arzneiwirkstoff enthaltenen Zusammensetzungen auf verschiedene Parameter stützt, die mit der Beschaffenheit des Arzneiwirkstoffes selbst verbunden sind. Ohne Einschränkung darauf sind Beispiele dieser Parameter die Wirkstabilität des Ausgangsstoffes unter verschiedenen Umgebungsbedingungen, die Stabilität im Verlauf der Herstellung der pharmazeutischen Formulierung sowie die Stabilität in den Endzusammensetzungen des Arzneimittels. Der zur Herstellung der vorgenannten Arzneimittelzusammensetzungen verwendete Arzneiwirkstoff sollte so rein wie möglich sein und seine Stabilität bei Langzeitlagerung muß unter verschiedenen Umgebungsbedingungen gewährleistet sein. Dies ist zwingend erforderlich, um zu verhindern, daß Arzneimittelzusammensetzungen Verwendung finden, in denen neben tatsächlichem Wirkstoff beispielsweise Abbauprodukte desselben enthalten sind. In einem solchen Fall könnte ein in späteren Darreichungsformen ' vorgefundener Gehalt an Wirkstoff niedriger sein als spezifiziert.

Ein weiterer für die Herstellung von festen Darreichungsformen bedeutsamer Aspekt ist darin zu sehen, daß für die pharmazeutische Qualität einer Arzneimittelformulierung eine möglichst stabile, kristalline Morphologie des Wirkstoffs gewährleistet sein sollte. Ist dies nicht der Fall, so kann es unter Umständen unter den Herstellungsbedingungen der Darreichungsform zu einer Veränderung der Morphologie des Wirkstoffs kommen. Eine solche Veränderung kann wiederum einen Einfluß auf die Reproduzierbarkeit des Herstellverfahrens haben und somit zu Endformulierungen führen, die den hohen Qualitätsanforderungen, die an Arzneimittelformulierungen zu richten sind, nicht genügen. Insofern ist generell zu berücksichtigen, dass jede Änderung des Feststoffzustandes eines Arzneimittels, welche dessen physikalische und chemische Stabilität verbessern kann, gegenüber weniger stabilen Formen desselben Arzneimittels einen erheblichen Vorteil ergibt.

Die Aufgabe der Erfindung besteht somit in der Bereitstellung einer neuen, stabilen Form des Telmisartans, die den vorstehend genannten hohen Anforderungen, die an einen Arzneimittelwirkstoff zu richten sind, genügen.

### Detaillierte Beschreibung der Erfindung

Es wurde überraschenderweise gefunden, daß Telmisartan in Form seines Natriumsalzes der Formel **1** 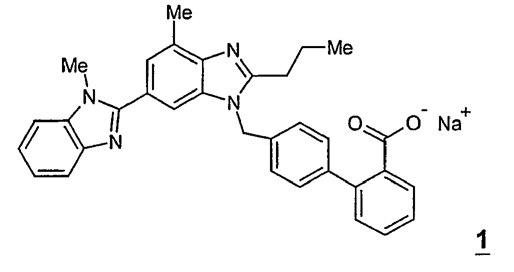 in kristalliner Form erhalten werden kann. Durch geeignete Wahl der Herstellungsbedingungen, kann dabei diejenige polymorphe Form des kristallinen Natriumsalzes, die den eingangs genannten Anforderungen genügt, in selektiver Art und Weise erhalten werden.

Diese kristalline Form des Telmisartan-natriumsalzes ist gekennzeichnet durch einen Schmelzpunkt von T=245 ± 5°C (bestimmt über DSC=Differential Scanning Calorimetry; Heizrate: 10 K/min).

Die vorliegende Erfindung betrifft daher kristallines Telmisartan-natriumsalz gekennzeichnet durch einen Schmelzpunkt von T=245 ± 5°C (bestimmt über DSC). Der vorstehende Wert wurde mittels eines DSC821 der Firma Mettler-Toledo erhoben.

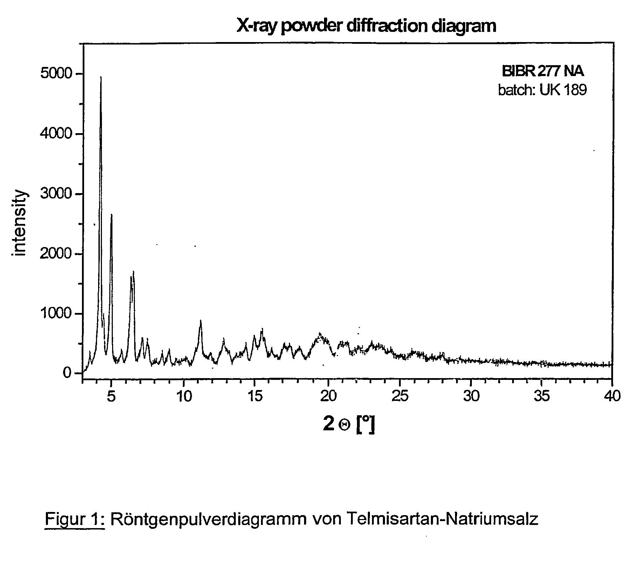

Die erfindungsgemäße kristalline Form des Telmisartan-Natriumsalzes wurde spektroskopisch näher untersucht. Das erhaltenen Röntgenpulverdiagramm ist in Figur 1 dargestellt.
Nachstehende Tabelle 1 fast die bei dieser sprektroskopischen Analyse erhaltenen Daten zusammen:

**Tabelle 1:**

| **2Θ [°]** | **d[Å]** | **rel. Intensität [%]** | | **2 Θ [°]** | **d [Å]** | **rel. Intensität [%]** |
|---|---|---|---|---|---|---|
| 3,54 | 24,96 | 7 | | 13,17 | 6,72 | 7 |
| 4,21 | 20,95 | 100 | | 13,68 | 6,47 | 7 |
| 4,45 | 19,83 | 20 | | 14,36 | 6,16 | 10 |
| 4,98 | 17,72 | 54 | | 14,98 | 5,91 | 13 |
| 5,69 | 15,52 | 8 | | 15,51 | 5,71 | 14 |
| 6,32 | 13,97 | 34 | | 15,70 | 5,64 | 12 |
| 6,48 | 13,63 | 35 | | 16,21 | 5,46 | 8 |
| 7,12 | 12,41 | 12 | | 17,09 | 5,18 | 10 |
| 7,49 | 11,80 | 11 | | 17,48 | 5,07 | 9 |
| 8,08 | 10,93 | 4 | | 18,10 | 4,90 | 9 |
| 8,49 | 10,41 | 6 | | 19,18 | 4,62 | 11 |
| 8,96 | 9,86 | 7 | | 19,43 | 4,56 | 13 |
| 9,50 | 9,31 | 5 | | 19,95 | 4,45 | 11 |
| 10,19 | 8,68 | 5 | | 20,89 | 4,25 | 11 |
| 10,80 | 8,18 | 8 | | 21,29 | 4,17 | 10 |
| 11,16 | 7,92 | 18 | | 22,19 | 4,00 | 9 |
| 11,88 | 7,44 | 7 | | 23,07 | 3,85 | 10 |
| 12,51 | 7,07 | 7 | | 23,76 | 3,74 | 9 |
| 12,79 | 6,92 | 11 | | 24,43 | 3,64 | 8 |

In vorstehender Tabelle steht der Wert "2 Θ [°]" für den Beugungswinkel in Grad und der Wert "d [Å]" für die bestimmten Gitterebenenabstände in Å.

Entsprechend den in Tabelle 1 dargestellten Befunden betrifft die vorliegende Erfindung kristallines Telmisartan-Natriumsalz, dadurch gekennzeichnet, daß es im Röntgenpulverdiagramm unter anderen die charakteristischen Werte d= 20,95 Å, 17,72 Å, 13,97 Å und 13,63 Å aufweist.

Die Röntgenpulverdiagramme wurden im Rahmen der vorliegenden Erfindung aufgenommen mittels eines Bruker D8 Advanced mit einem OED (= ortsempfindlicher Detektor) (CuK_{α} - Strahlung, λ, = 1.5418 Å, 30 kV, 40 mA).

Die vorliegende Erfindung betrifft ferner das erfindungsgemäße kristalline Telimsartan-Natriumsalz in Form seiner Solvate und Hydrate, bevorzugt in Form seiner Hydrate, besonders bevorzugt in Form seines Hemihydrats.

Ein weiterer Aspekt der vorliegenden Erfindung betrifft ein Herstellverfahren zur Darstellung des erfindungsgemäßen kristallinen Telmisartan-Natriumsalzes.
Als Ausgangsmaterial zur Darstellung von erfindungsgemäßem kristallinem Telmisartan-Natriumsalz kann die freie Säure Telmisartan dienen, die nach im Stand der Technik bekannten Verfahren (z.B. gemäß EP 502314 A1) erhalten werden kann.

Zur Darstellung des erfindungsgemäßen kristallinen Natriumsalzes wird die freie Säure des Telmisartans in einem geeigneten Lösemittel, vorzugsweise in einem organischen aprotischen Lösemittel, besonders bevorzugt in einem organischen, aprotischen und unpolaren Lösemittel aufgenommen. Als Lösemittel kommen erfindungsgemäß besonders bevorzugt in Betracht Toluol, Chloroform, Dichlormethan, Tetrahydrofuran, Diethylether, Diisopropylether, Methyl-tertiärbutylether, Aceton, Methylisobutylketon, Benzol oder Acetonitril, wobei Toluol Benzol und Methylisobutylketon besonders bevorzugt sind. Erfindungsgemäß von herausragender Bedeutung ist als Lösemittel Toluol.
Pro Gramm Telmisartan (freie Säure) gelangen bevorzugt zwischen 0,5 und 5 ml, bevorzugt zwischen 1 und 3 ml, besonders bevorzugt zwischen 1,5 und 2,5 ml des vorstehend genannten Lösemittels zur Anwendung.
Zu dieser Lösung oder Suspension wird anschließend ein geeignetes Natriumsalz als Base zugesetzt. Als geeignete Natriumsalze kommen im Rahmen der vorliegenden Erfindung Natriumhydroxid, Natriumhydrid, Natriumcarbonat, Natriumhydrogencarbonat oder Natriumalkoholate in Betracht. Unter Natriumalkoholaten werden die Natriumsalze verstanden, die mit niederen
Alkoholen, vorzugsweise mit Alkoholen ausgewählt aus der Gruppe bestehend aus Methanol, Ethanol, Isopropanol, n-Propanol, tert-Butanol, sec.-Butänol, isoButanol,n-Butanol und tert.-Amylalkohol, gebildet werden. Erfindungsgemäß von besonderem Interesse sind Natriumsalze ausgewählt aus der Gruppe bestehend aus Natriumhydroxid, Natriumhydrid, Natriumethanolat und Natriummethanolat, wobei das Natriumhydroxid und das Natriummethanolat von erfindungsgemäß herausragender Bedeutung ist. Die vorstehend genannten Natriumsalze können der Reaktionsmischung als Feststoffe zugesetzt werden. Im Falle des Natriumhydroxids ist die Zugabe in Form wässeriger Lösungen allerdings bevorzugt. Hierbei werden besonders bevorzugt konzentrierte wässrige Lösungen des Natriumhydroxids verwendet. Beispielsweise kann Natronlage mit einer Konzentration von etwa 45 Gew.-% zum Einsatz gelangen.
Die Menge an einzusetzendem Natriumsalz ist naturgemäß abhängig von der Menge an eingesetzter freier Säure Temisartan. Erfindungsgemäß müssen pro Mol Telmisartan wenigstens 1 Mol Natriumsalz zugesetzt werden. Eine Zugabe an Natriumsalz im Überschuß ist erfindungsgemäß ebenfalls möglich. Bevorzugt werden pro Mol eingesetzte Säure Telmisartan 1-2,5, bevorzugt 1-2, besonders bevorzugt 1-1,5 Mol Natriumsalz zugesetzt.
Wird als Natriumsalz Natriumhydroxid verwendet und dieses gemäß einer bevorzugten Ausführungsform des erfindungsgemäßen Verfahrens in Form wässeriger Lösungen zugesetzt, kann es gegebenenfalls hilfreich sein, ein mit Wasser mischbares organisches Lösemittel zuzusetzen. Dieses ist vorzugsweise ausgewählt aus der Gruppe bestehend aus Methanol, Ethanol, Isopropanol, Aceton, Tetrahydrofuran, tert.-Butanol, 2-Butanol, Butanol,Glycol, Ethyldiglycol, 1,3-Butandiol, 1,4-Butandiol, tert.-Amylalkohol, Acetonitril, Nitromethan, Formamid, Dimethylformamid, N-Methylpyrrolidinon, Dimethylsulfoxid, Dimethylacetamid, Nitroethan, Methoxy-2-Propanol, wobei den vorstehend genannten Alkoholen eine besondere Bedeutung zukommt. Besonders bevorzugt werden im Rahmen des erfindungsgemäßen Verfahrens Methanol oder Ethanol, besonders bevorzugt Ethanol verwendet. Pro Mol eingesetztes Telmisartan werden erfindungsgemäß bevorzugt zwischen 50 und 500 ml, besonders bevorzugt zwischen 100 und 400 ml, ferner bevorzugt zwischen 200 und 350 ml dieses Lösemittels verwendet. Anschließend kann die Reaktionsmischung erwärmt und damit das Fortschreiten der Reaktion beschleunigt werden. Bevorzugt wird die Reaktionsmischung unter guter Durchmischung auf eine Temperatur von >40°C, besonders bevorzugt auf über 60°C erwärmt. Die maximal wählbare Temperatur bestimmt sich natürlich durch die Siedetemperatur der verwendeten Lösemittel. Werden die erfindungsgemäß gemäß vorstehender Ausführungen bevorzugten Lösemittel eingesetzt, wird bevorzugt auf über 70°C erwärmt. Diese Erwärmung wird in der Regel für einen Zeitraum von 15 Minuten bis 2 Stunden, bevorzugt zwischen 20 Minuten und einer Stunde durchgeführt. Anschließend wird die erhaltene Lösung filtriert und gegebenenfalls im Filter verbleibender Feststoff mit einem oder mehreren der vorstehend genannten Lösemittel nachgewaschen.

Zu einem auf eine Temperatur von >40°C, bevorzugt auf über 60°C, besonders bevorzugt bis zur Siedehitze erwärmten organischen Lösemittel, wird das gemäß vorstehend beschriebener Vorgehensweise erhaltene Filtrat langsam, vorzugsweise tropfenweise zugegeben. Als Lösemittel kommt an dieser Stelle vorzugsweise ein organisches aprotisches Lösemittel, besonders bevorzugt ein organisches, aprotisches und unpolares Lösemittel in Betracht. Als Lösemittel kommen erfindungsgemäß besonders bevorzugt in Betracht Toluol, Chloroform, Dichlormethan, Tetrahydrofuran, Diethylether, Diisopropylether, Methyl-tertiärbutylether, Aceton, Methylisobutylketon, Benzol oder Acetonitril, wobei Toluol, Benzol und Methylisobutylketon besonders bevorzugt sind. Erfindungsgemäß von herausragender Bedeutung ist an dieser Stelle das Lösemittel Toluol. Gleichzeitig werden während der Zugabe des Filtrats zu dem vorgelegten erwärmten Lösemittel bei einer bevorzugten Ausführungsform des Verfahrens Teile des Lösemittels (gegebenenfalls azeotrop) abdestilliert. Nach beendeter Zugabe wird gegebenenfalls weiteres Lösemittel (beispielhaft etwa 1-2 Drittel der bis zu diesem Zeitpunkt insgesamt eingesetzten Lösemittelmenge) destillativ entfernt.

Die so erhaltene konzentrierte Lösung wird abgekühlt, vorzugsweise auf Raumtemperatur, wobei Telmisartan-Natriumsalz auskristallisiert. Nach beendeter Kristallisation werden die Kristalle abgetrennt, gegebenenfalls mit dem eingangs genannten organischen Lösemittel gewaschen und abschließend getrocknet.

In einer weiteren Ausführungsform der Erfindung ist das erfindungsgemäße kristalline Telmisartan-Natriumsalz ausgehend von den Säureadditionssalzen der Formel **2** 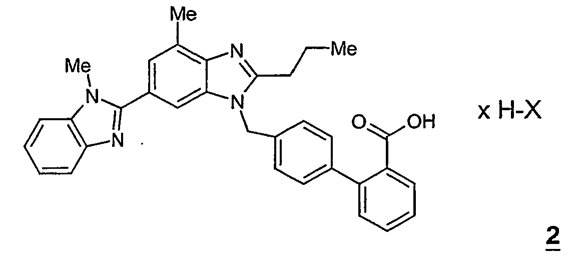 worin H-X für eine Säure ausgewählt aus der Gruppe bestehend aus Salzsäure, Bromwasserstoffsäure, Toluolsulfonsäure oder Methansulfonsäure steht, erhältlich. Von den vorstehend genannten Säureadditionssalzen der Formel **2** kommt dem Salz, in dem H-X für Salzsäure steht, besondere Bedeutung zu. Nachfolgend wird dieses Säureadditionssalz auch als Telmisartan-hydrochlorid bezeichnet.

Die Verbindungen der Formel **2** sind beispielsweise aus dem aus dem Stand der Technik bekannten 4'-[[2-n-Propyl-4-methyl-6-(1-methylbenzimidazol-2-yl)-benzimidazol-1-yl]-methyl]-biphenyl-2-carbonsäure-*tert*.-butylester (= Telmisartan-tert.-butylester) durch Verseifung in Essigsäure in Gegenwart der Säure H-X erhältlich.

Zur Darstellung des erfindungsgemäßen kristallinen Telmisartan-natriumsalzes der Formel **1** ausgehend von den Säureadditionssalzen der Formel **2** kann erfindungsgemäß wie folgt vorgegangen werden.

Die Verbindung der Formel **2** wird in einem geeigneten Lösemittel aufgenommen und mit einem geeigneten Natriumsalz versetzt.
Als Lösemittel kommen Wasser und/oder ein geeigneter Alkohol, wie Methanol, Ethanol oder Isopropanol im Gemisch mit einem aprotischen organischen Lösmittel ausgewählt aus der Gruppe bestehend aus Toluol, Chloroform, Dichlormethan, Tetrahydrofuran, Diethylether, Diisopropylether, Methyl-tertiärbutylether, Aceton, Methylisobutylketon, Benzol und Acetonitril in Betracht. Besonders bevorzugt wird als Lösmittel Wasser im Gemisch mit Ethanol oder lsopropanol im Gemisch mit einem aprotischen organischen Lösemittel ausgewählt aus der Gruppe bestehend aus Toluol, Benzol und Methylisobutylketon, besonders bevorzugt Toluol, verwendet. Als besonders geeignet hat sich für diesen Syntheseschritt ein Gemisch aus Wasser, Isopropanol und Toluol erwiesen.
Die Menge an eingesetztem Lösemittel bzw. Lösemittelgemisch ist abhängig von der Menge an eingesetztem Säureadditionssalz **2**. Vorzugsweise werden pro Mol eingesetzter Verbindung **2** etwa 0,3 - 3,5 L, bevorzugt etwa 1 - 2,5 L, besonders bevorzugt etwa 1,5 - 2 L des vorstehend genannten Lösemittels bzw. Lösemittelgemischs verwendet. Gelangt als Lösemittel jenes erfindungsgemäß bevorzugte Lösemittelgemisch zur Anwendung, welches neben Wasser und einem aprotischen organischen Lösemittel ferner einen Alkohol als dritte Lösemittelkomponente enthält, liegen die volumenbezogenen Verhältnisse von Wasser zum aprotischen organischen Lösemittel erfindungsgemäß bevorzugt in einem Bereich von 1:5 bis 1:50 und die von Wasser zum eingesetzten Alkohol in einem Verhöltnis von 2:1 bis 1:40. Bevorzugt liegen in einem solchen Lösmittelgemisch die Verhältnisse von Wasser zum aprotischen organischen Lösemittel in einem Bereich von 1:10 bis 1:30, bevorzugt in einem Bereich von 1:15 bis 1:25 und die von Wasser zum eingesetzten Alkohol in einem Verhältnis von 1:1 bis 1:20, bevorzugt in einem Bereich von 1:5 bis 1:15.
Vorzugsweise enthält das vorstehend genannte Lösemittel bzw. Lösemittelgemisch pro Mol 2 etwa 10 bis 100 ml Wasser, bevorzugt etwa 30 bis 80 ml Wasser, besonders bevorzugt etwa 40 bis 70 ml Wasser. Vorzugsweise enthält das eingesetzte Lösemittel bzw. Lösemittelgemisch pro Mol 2 ferner etwa 100 bis 1000 ml Alkohol, bevorzugt etwa 300 bis 800 ml Alkohol, besonders bevorzugt etwa 400 bis 700 ml Alkohol. Schließlich enthält das eingesetzte Lösemittel bzw. Lösemittelgemisch pro Mol **2** vorzugsweise als dritte Lösemittelkomponente etwa 200 bis 2000 ml des vorstehend genannten aprotischen organischen Lösemittels, bevorzugt etwa 600 bis 1600 ml, besonders bevorzugt etwa 800 bis 1400 ml des vorstehend genannten aprotischen organischen Lösemittels.

Als geeignete Natriumsalze kommen für die Umsetzung von **2** zu **1** Natriumhydroxid, Natriumhydrid, Natriumcarbonat, Natriumhydrogencarbonat oder Natriumalkoholate in Betracht. Unter Natriumalkoholaten werden die Natriumsalze verstanden, die mit niederen Alkoholen, vorzugsweise mit Alkoholen ausgewählt aus der Gruppe bestehend aus Methanol, Ethanol, Isopropanol, n-Propanol, tert-Butanol, sec.-Butanol, iso-Butanol,n-Butanol und tert.-Amylalkohol, gebildet werden. Erfindungsgemäß von besonderem Interesse sind Natriumsalze ausgewählt aus der Gruppe bestehend aus Natriumhydroxid, Natriumhydrid, Natriumethanolat und Natriummethanolat, wobei die Natriumalkoholate Natriumethanolat und Natriummethanolat, insbesondere Natriummethanolat für diesen Reaktionsschritt von erfindungsgemäß herausragender Bedeutung ist. Die vorstehend genannten Natriumsalze können der Reaktionsmischung als Feststoffe zugesetzt werden. Im Falle des Natriummethanolats ist die Zugabe in Form methanolischer Lösungen allerdings bevorzugt. Hierbei werden besonders bevorzugt methanolische Lösungen des Natriummethanolats verwendet, die diese in einer Konzentration von wenigstens 10%, besonders bevorzugt von etwa 20-40 % (w/w) enthalten. Beispielsweise kann die methanolische Natriummethanolatlösung mit einer Konzentration von etwa 30 Gew.-% zum Einsatz gelangen.
Die Menge an einzusetzendem Natriumsalz ist naturgemäß abhängig von der Menge an eingesetzter freier Säure Temisartan. Erfindungsgemäß müssen pro Mol eingesetztes Telmisartan-Säureadditionssalz der Formel **2** wenigstens 2 Mol Natriumsalz zugesetzt werden. Eine Zugabe an Natriumsalz im Überschuß ist erfindungsgemäß ebenfalls möglich.

Gegebenenfalls kann es sinnvoll erscheinen, der vorstehend genannten Reaktionsmischung Aktivkohle zuzusetzen. Beispielsweise kann dies in einer Menge von etwa 5 - 50 g pro Mol eingesetztes **2**, vorzugsweise in einer Menge von etwa 10 - 40 g pro Mol eingesetztes 2 erfolgen.
Nach erfolgter Zugabe des Natriumsalzes sowie gegebenenfalls erfolgte Zugabe von Aktivkohle wird das erhaltene Reaktionsgemisch für einen Zeitraum von etwa 10 Minuten bis 2 Stunden, vorzugsweise für etwa 20-45 Minuten auf eine Temperatur von etwa 50-100°C, vorzugsweise auf etwa 60-90°C, besonders bevorzugt auf etwa 70-80°C erwärmt. Im Zuge dieser Erwärmung kann ein Teil des Lösemittels, vorzugsweise etwa 10-50%, besondersbevorzugt etwa 20-40% der Gesamtlösemittelmenge abdestilliert werden.

Die verbleibende Suspension wird anschließend filtriert, der Filterrückstand gegebenenfalls mit einem der vorstehend genannten aprotischen organischen Lösemittel, vorzugsweise mit jenem aprotischen organischen Lösmittel, welches auch zur Durchführung der Reaktion Verwendung findet, gewaschen.
Das erhaltene Filtrat wird sodann mit einem Lösemittel bzw. Lösemittelgemisch verdünnt. Hierbei wird vorzugsweise eine Gemisch aus Wasser und dem vorstehend genannten aprotischen organischen Lösemittel verwendet. Vorzugsweise werden an dieser Stelle pro Mol ursprünglich eingesetzter Verbindung **2** etwa 10 bis 100 ml Wasser, bevorzugt etwa 30 bis 80 ml Wasser, besonders bevorzugt etwa 40 bis 70 ml Wasser verwendet. An aprotischem organischen Lösemittel werden an dieser Stelle vorzugsweise pro Mol ursprünglich eingesetzter Verbindung **2** 250 bis 3000 ml, bevorzugt etwa 800 bis 2000 ml, besonders bevorzugt etwa 1200 bis 1800 ml verwendet.

Nach Verdünnung wird die erhaltene Mischung bis zum Rückfluß erwärmt. Dabei werden pro Mol ursprünglich eingesetzter Verbindung **2** etwa 1-2 L, vorzugsweise etwa 1200 bis 1800 ml Lösemittel abdestilliert. Nach Abdestillieren des Lösemittels kristallisiert das erfindungsgemäße Telmisartan-Natriumsalz **1** aus. Die erhaltenen Kristalle werden isoliert, gegebenenfalls mit einem der vorstehend genannten aprotischen, organischen Lösemittel gewaschen und abschließend getrocknet.

Ein weiterer Aspekt der vorliegenden Erfindung betrifft kristallines Telmisartan-Natriumsalz, gegebenenfalls in Form seiner Solvate oder Hydrate, bevorzugt in Form seiner Hydrate, besonders bevorzugt in Form seines Hemihydrats, welches gemäß vorstehend beschriebener Vorgehensweisen erhältlich ist.

Aufgrund der zentralen Bedeutung der Verbindungen der Formel **2** als wertvolle Ausgangsmaterialien zur direkten Synthese des erfindungsgemäßen Telmisartan-Natriumsalzes **1** betrifft ein weiterer Aspekt der vorliegenden Erfindung, Verbindungen der Formel **2** als solche 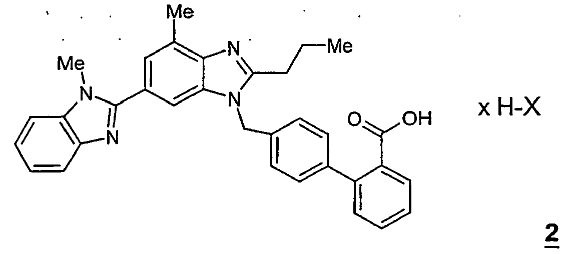 worin H-X für Toluolsulfonsäure oder Methansulfonsäure steht.

Besonders bevorzugt betrifft die vorliegende Erfindung ferner die vorstehend genannten Verbindungen der Formel **2** in kristalliner Form.

Ferner betrifft die vorliegende Erfindung aufgrund der pharmazeutischen Wirksamkeit des erfindungsgemäßen kristallinen Telmisartan-Natriumsalzes dessen Verwendung als Arzneimittel.

Ein weiterer Aspekt der vorliegenden Erfindung betrifft aufgrund der pharmazeutischen Wirksamkeit des erfindungsgemäßen kristallinen Telmisartan-Natriumsalzes dessen Verwendung zur Herstellung eines Arzneimittels, insbesondere zur Herstellung eines Arzneimittels zur Vorbeugung oder Behandlung von Erkrankungen, in denen die Applikation therapeutisch wirksamer Dosen eines oder mehrerer Angiotensin-II-Antagonisten einen therapeutischen Nutzen entfalten kann. Bevorzugt zielt die vorliegende Erfindung auf die Verwendung von kristallinem Telmisartan-Natriumsalz zur Herstellung eines Arzneimittels zur Vorbeugung oder Behandlung von Erkrankungen ausgewählt aus der Gruppe bestehend aus Hypertonie, Herzinsuffizienz, ischämische periphere Durchblutungsstörungen, myokardiale lschämie (Angina), Herzinsuffizienzprogression nach Myokard-Infarkt, diabetische Neuropathie, Glaukom, gastrointestinale Erkrankungen und Blasenerkrankungen, wobei die Vorbeugung oder Behandlung von Hypertonie besonders bevorzugt ist.

Dementsprechend zielt ein weiterer Aspekt der vorliegenden Erfindung auf pharmazeutische Formulierungen gekennzeichnet durch einen Gehalt von kristallinem Telmisartan-Natriumsalz.

Das nachfolgende Synthesebeispiel dient der Illustration eines exemplarisch durchgeführter Herstellungsverfahrens für kristallines Telmisartan-Natriumsalz. Es ist lediglich als mögliche, exemplarisch dargestellte Vorgehensweise zu verstehen, ohne die Erfindung auf dessen Inhalt zu beschränken.

### Synthesebeispiel 1: Darstellung von kristallinem Telmisartan-natriumsalz ausgehend von Telmisartan:

Als Ausgangsmaterial zur Darstellung von erfindungsgemäßem kristallinem Telmisartan-Natriumsalz kann die freie Säure Telmisartan dienen, die nach im Stand der Technik bekannten Verfahren (z.B. gemäß EP 502314 A1) erhalten werden kann.
In einem geeigneten Reaktionsgefäß werden 154,4 g Telmisartan in 308,8 ml Toluol vorgelegt. Die Suspension wird mit 27,8 g 44,68%iger Natronlauge und 84,9 ml Ethanol versetzt und auf 78°C etwa 30 min erwärmt, der Ansatz anschließend filtriert. Gegebenenfalls kann bei Verbleib größerer Feststoffmengen im Filter mit einem Gemisch aus 61,8 ml Toluol und 15,3 ml Ethanol nachgewaschen werden.

In einem weiteren Reaktionsgefäß werden 463,2 ml Toluol vorgelegt und zum Rückfluß erhitzt. Man tropft in der Siedehitze das gemäß vorstehend beschriebener Vorgehensweise erhältliche Filtrat langsam zu und destilliert gleichzeitig azeotrop ab.

Nach Beendigung der Zugabe kann die gegebenenfalls aus der Nachwaschung des Filters erhaltene Lösung ebenfalls zudosiert und auch hier dabei azeotrop abdestilliert werden. Man destilliert bis 103°C und läßt die Suspension auf Raumtemp. abkühlen. Die Kristalle werden abgesaugt, mit 154,4 ml Toluol gewaschen und im Umlufttrockenschrank bei 60°C getrocknet.

Ausbeute: 154,6 g (96%), farblose Kristalle;

| | | | |
|---|---|---|---|
| C₃₃H₂₉N₄O₂Na x 0,5H₂O | ber.: C 72,51 | H 5,72 | N 10,25 |
| | gef.: C 72,57 | H 5,69 | N 10,21 |

### Synthesebeispiel 2: Darstellung von kristallinem Telmisartan-natriumsalz ausgehend von Telmisartan-hydrochlorid:

### A) Darstellung von Telmisartan-hydrochlorid:

411 g 4'-[[2-n-Propyl-4-methyl-6-(1-methylbenzimidazol-2-yl)-benzimidazol-1-yl]-methyl]-biphenyl-2-carbonsäure-*tert*.-butylester werden in 822 ml Eisessig suspendiert und mit 213 g konzentrierter wässeriger Salzsäure (37%ig) versetzt. Man erhitzt die Mischung zum Rückfluß und destilliert etwa 640 ml Lösemittel ab.
Der verbleibende Rückstand wird bei 50-60°C langsam mit etwa 620 ml Wasser versetzt. Zu dieser Mischung werden 20 g Aktivkohle (z.B. Norit SX 2 Ultra) gegeben und die erhaltene Mischung etwa 10 min bei konstanter Temperatur gerührt. Nach Abfiltrieren wird der Rückstand mit dreimal je 25 ml Eisessig und mit etwa 620 ml Wasser gewaschen. Das erhaltene Filtrat wird erneut auf etwa 50-60°C erwärmt und mit etwa 2 L Wasser versetzt. Nach Rühren über etwa 12 Stunden bei etwa 23°C werden die entstandenen Kristalle abgesaugt und zweimal mit etwa 500 ml Wasser, einmal mit etwa 900 ml Aceton gewaschen und anschließend bei etwa 60°C getrocknet.

Ausbeute: 367 g (92,5%), farblose Kristalle, Schmp.: = 278°C

### B) Darstellung kristallines Telmisartan-Natriumsalz aus Telmisartan-hydrochlorid

Es werden 55,1 g Telmisartan-hydrochlorid in 110,2 ml Toluol, 5,5 ml Wasser, 55,1 ml iso-Propanol aufgenommen und versetzt diese Mischung mit 36,9 g Natriummethylat (30%ig in Methanol) und 2,75 g Aktivkohle (z.B. Sorit SX 2 Ultra). Anschließend wird auf etwa 75°C erwärmt, und bei konstanter Temperatur über etwa 30 min ca. 50 ml Lösungsmittelgemisch abdestilliert. Die erhaltene Suspension wird filtriert und der Rückstand mit etwa 20 ml Toluol gewaschen. Das Filtrat wird mit etwa 5 ml Wasser und etwa 150 ml Toluol versetzt. Die erhaltene Mischung wird zum Rückfluß erhitzt. Dabei werden etwa 150 ml Lösemittelgemisch azeotrop abdestilliert (bei bis zu 102°C). Man läßt eine Stunde bei 100°C durchkristallisieren. Die Kristalle werden abgesaugt, mit etwa 50 ml Toluol gewaschen und bei ca. 60°C getrocknet.

| | | | |
|---|---|---|---|
| Ausbeute: 53,6 g (99%), farblose Kristalle ; | | | |
| C₃₃H₂₉N₄O₂Na·0,5H₂O | ber.: C 72,51 | H 5,72 | N 10,25 |
| | gef.:C 72,44 | H 5,68 | N 10,20 |

Zur Herstellung eines den Wirkstoff enthaltenden Arzneimittels, insbesondere eines oral applizierbaren Arzneimittels, besonders bevorzugt einer Tablette, kann nach im Stand der Technik bekannten Verfahren vorgegangen werden.
Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Im Folgenden sind einige Beispiele für erfindungsgemäß einsetzbare pharmazeutische Zubereitungen angegeben. Diese dienen lediglich der beispielhaften Erläuterung, ohne den Gegenstand der Erfindung auf selbige zu beschränken.

**Tablette 1:**

| Bestandteile: | mg |
|---|---|
| Telmisartan-Natriumsalz-hemihydrat | 1,00 |
| Mannitol | 121,50 |
| Maisstärke | 79,85 |
| Hochdisperses Siliciumdioxid, wasserfrei | 2,30 |
| Polyvidon K25 | 2,35 |
| Magnesiumstearat | 3,00 |
| Gesamt | 210,00 |

**Tablette 2:**

| Bestandteile: | mg |
|---|---|
| Telmisartan-Natriumsalz-hemihydrat | 0,5 |
| Mannitol | 122,0 |
| Maisstärke, getrocknet | 61,8 |
| Maisstärke | 18,0 |
| Hochdisperses Siliciumdioxid, wasserfrei | 2,4 |
| Polyvidon K25 | 2,3 |
| Magnesiumstearat | 3,0 |
| gesamt | 210,0 |

**Tablette 3:**

| Bestandteile: | mg |
|---|---|
| Telmisartan-Natriumsalz-hemihydrat | 0,25 |
| Mannitol | 61,00 |
| Maisstärke | 39,90 |
| Hochdisperses Siliciumdioxid, wasserfrei | 1,20 |
| Polyvidon K25 | 1,15 |
| Magnesiumstearat | 1,5 |
| Gesamt | 105,00 |

## Patentansprüche

1. Kristallines Telmisartan-natriumsalz der Formel **1** 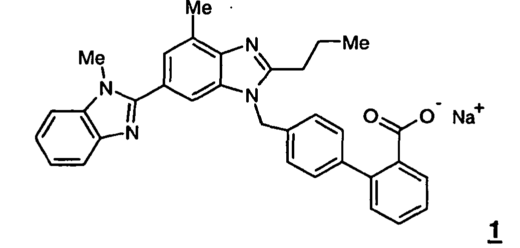 **gekennzeichnet durch** einen Schmelzpunkt von T=245 ± 5°C.

2. Kristallines Telmisartan-Natriumsalz nach Anspruch 1, **dadurch gekennzeichnet, daß** es im Röntgenpulverdiagramm unter anderen die charakteristischen Werte d= 20,95 Å, 17,72 Å, 13,97 Å und 13,63 Å aufweist.

3. Solvat, Hydrat oder Hemihydrat des kristallinen Telmisartan Natriumsalzes nach einem der Ansprüche 1 oder 2,

4. Verfahren zur Herstellung von kristallinem Telmisartan-Natriumsalz **1**, **dadurch gekennzeichnet, daß** die freie Säure des Telmisartans in einem geeigneten Lösemittel aufgenommen wird, diese Lösung anschließend mit einem geeigneten Natriumsalz versetzt wird, wobei pro Mol Telmisartan wenigstens 1 Mol Natriumsalz zugesetzt wird, anschließend die Reaktionsmischung über einen Zeitraum von 15 Minuten bis 2 Stunden erwärmt wird, anschließend die erhaltene Lösung filtriert wird und das so erhaltene Filtrat zu einem auf eine Temperatur von >40°C erwärmten organischen Lösemittel langsam zugegeben wird, Teile des Lösemittels gegebenenfalls bereits während der Zugabe vorstehend genannten Filtrats abdestilliert werden, die so erhaltene konzentrierte Lösung anschließend abgekühlt wird und das dabei auskristallisierende Telmisartan-Natriumsalz isoliert und, gegebenenfalls nach Waschung mit dem eingangs genannten organischen Lösemittel, getrocknet wird.

5. Verfahren zur Herstellung von kristallinem Telmisartan-Natriumsalz **1**, **dadurch gekennzeichnet, daß** ein Säureadditionssalz der Formel **2** 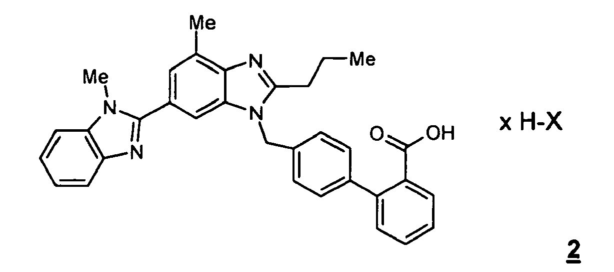 worin H-X für eine Säure ausgewählt aus der Gruppe bestehend aus Salzsäure, Bromwasserstoffsäure, Toluolsulfonsäure oder Methansulfonsäure steht, in einem geeigneten Lösemittel aufgenommen und mit einem geeigneten Natriumsalz umgesetzt wird, wobei pro Mol eingesetzte Verbindung 2 wenigstens 2 Mol Natriumsalz zum Einsatz gelangen.

6. Kristallines Telmisartan-Natriumsalz, erhältlich nach Anspruch 4 oder 5.

7. Verwendung von kristallinen Telmisartan-Natriumsalz gemäß einem der Ansprüche 1-3 oder 6 zur Herstellung eines Arzneimittels.

8. Pharmazeutische Formulierungen **gekennzeichnet durch** einen Gehalt an kristallinem Telmisartan-Natriumsalz gemäß einem der Ansprüche 1-3 oder 6.

9. Verbindungen der Formel **2** 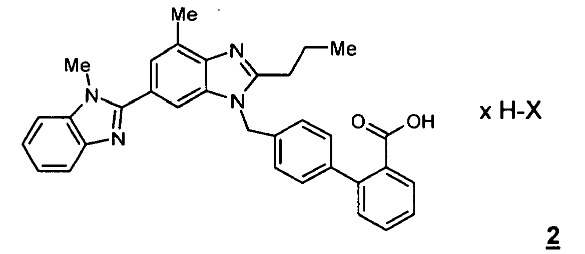 worin H-X für Toluolsulfonsäure oder Methansulfonsäure steht.

10. Verbindung nach Anspruch 9, **dadurch gekennzeichnet, daß** sie in kristalliner Form vorliegt.

## Claims

1. Crystalline telmisartan-sodium salt of formula 1 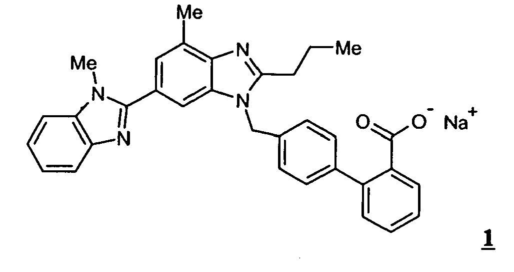 **characterised by** a melting point of T=245 ± 5°C.

2. Crystalline telmisartan-sodium salt according to claim 1, **characterised in that** in the X-ray powder diagram it has, *inter alia,* the characteristic values d= 20.95 Å, 17.72 Å, 13.97 Å and 13.63 Å.

3. Solvate, hydrate or hemihydrate of the crystalline telmisartan-sodium salt according to one of claims 1 or 2.

4. Process for preparing crystalline telmisartan-sodium salt **1**, **characterised in that** the free acid of telmisartan is taken up in a suitable solvent, this solution is then combined with a suitable sodium salt, at least 1 mol of sodium salt being added per mol of telmisartan, then the reaction mixture is heated for a period of from 15 minutes to 2 hours, then the solution obtained is filtered and the resulting filtrate is slowly added to an organic solvent heated to a temperature of >40°C, some of the solvent is optionally distilled off even while the abovementioned filtrate is being added, the resulting concentrated solution is then cooled and the telmisartan-sodium salt which crystallises out is isolated and dried, optionally after washing with the organic solvent mentioned hereinbefore.

5. Process for preparing crystalline telmisartan-sodium salt **1, characterised in that** an acid addition salt of formula **2** 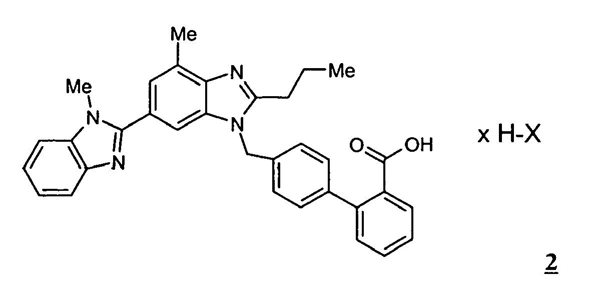 wherein H-X denotes an acid selected from among hydrochloric acid, hydrobromic acid, toluenesulphonic acid or methanesulphonic acid, is taken up in a suitable solvent and reacted with a suitable sodium salt, using at least 2 mol of sodium salt per mol of the compound **2** used.

6. Crystalline telmisartan-sodium salt which may be obtained according to claim 4 or 5.

7. Use of crystalline telmisartan-sodium salt according to one of claims 1-3 or 6 for preparing a medicament.

8. Pharmaceutical formulations, **characterised in that** they contain crystalline telmisartan-sodium salt according to one of claims 1-3 or 6.

9. Compounds of formula **2** 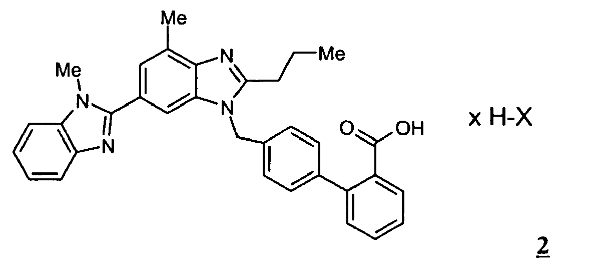 wherein H-X denotes toluenesulphonic acid or methanesulphonic acid.

10. Compound according to claim 9, **characterised in that** it is present in crystalline form.

## Revendications

1. Sel de sodium de telmisartan cristallin de formule 1 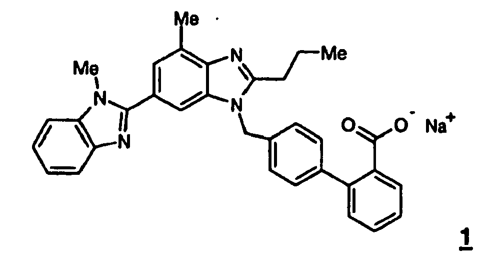 **caractérisé par** un point de fusion de T = 245 ± 5°C.

2. Sel de sodium de telmisartan cristallin selon la revendication 1 **caractérisé en ce qu'**il présente dans le diagramme de rayons X de poudre entre autres les valeurs caractéristiques d = 20,95 Å, 17,72 Å, 13,97 Å et 13,63 Å.

3. Solvate, hydrate ou hémihydrate du sel de sodium de telmisartan cristallin selon l'une des revendications 1 ou 2.

4. Procédé de production de sel de sodium de telmisartan cristallin **1 caractérisé en ce que** l'acide libre du telmisartan est repris dans un solvant approprié, cette solution est ensuite additionnée d'un sel de sodium approprié, où par mole de telmisartan au moins 1 mole de sel de sodium est ajoutée, puis le mélange réactionnel est chauffé pendant une durée de 15 minutes à 2 heures, ensuite la solution obtenue est filtrée et le filtrat ainsi obtenu est ajouté lentement à un solvant organique chauffé à une température >40°C, des parties du solvant sont chassées par distillation éventuellement dès au cours de l'addition du filtrat cité précédemment, la solution concentrée ainsi obtenue est ensuite refroidie et le sel de sodium de telmisartan qui cristallise alors est isolé et séché, éventuellement après lavage avec le solvant organique cité au début.

5. Procédé de production de sel de sodium de telmisartan cristallin **1 caractérisé en ce qu'**un sel d'addition d'acide de formule 2 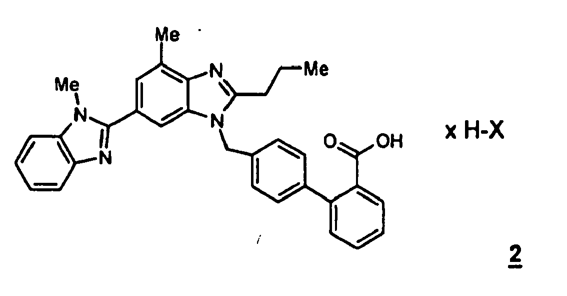 où H-X représente un acide choisi dans le groupe consistant en l'acide chlorhydrique, l'acide bromhydrique, l'acide toluènesulfonique ou l'acide méthanesulfonique, est repris dans un solvant approprié et mis à réagir avec un sel de sodium approprié, où par mole de composé **2** utilisé au moins 2 moles de sel de sodium sont utilisées.

6. Sel de sodium de telmisartan cristallin pouvant être obtenu selon la revendication 4 ou 5.

7. Utilisation du sel de sodium de telmisartan cristallin selon l'une des revendications 1-3 ou 6 pour la production d'un médicament.

8. Formulations pharmaceutiques **caractérisées par** une teneur en sel de sodium de telmisartan cristallin selon l'une des revendications 1-3 ou 6.

9. Composés de formule **2** 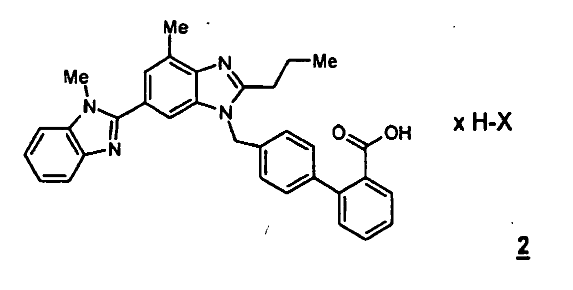 où H-X représente l'acide toluènesulfonique ou l'acide méthanesulfonique.

10. Composé selon la revendication 9 **caractérisé en ce qu'**il est sous forme cristalline.
